# EUROPEAN PATENT SPECIFICATION

(11) **EP 4 026 522 B1**
(45) Date of publication and mention of the grant of the patent: **30.09.2026**
(21) Application number: 20860860.4
(22) Date of filing: 16.06.2020
(51) Int. Cl.: A61F 2/66, A61F 2/60

(54) **SOLE OF ATHLETIC PROSTHETIC LEG**
SOHLE EINER SPORTPROTHESE
SEMELLE DE PROTHÈSE SPORTIVE

(30) Priority: 02.09.2019 JP 2019159849
(43) Date of publication of application: 13.07.2022
(73) Proprietor: BRIDGESTONE CORPORATION, Chuo-ku Tokyo 104-8340 (JP)
(72) Inventor: ITOI, Dyta, Tokyo 104-8340 (JP)
(74) Representative: Marks & Clerk LLP
(86) International application number: PCT/JP2020/023654
(87) International publication number: WO 2021/044702

(56) References cited:
- EP-A1- 1 972 223
- WO-A1-2017/013702
- US-A- 4 550 510
- US-A1- 2002 116 072
- US-A1- 2017 281 372
- US-B1- 8 535 390
- US-S- D 814 160
- MILES STUART: "Nike and Sarah Reinersten create new shoe tech for amputee athletes", 16 April 2013 (2013-04-16), XP093067704, Retrieved from the Internet <URL:https%3A%2F%2Fwww.pocket-lint.com%2Fgadgets%2Fnews%2Fnike%2F115074-sarah-reinstern-talks-nike-sole%2F> [retrieved on 20230726]
- BRIDGESTONE CORPORATION: "Supporting Athletes with Newly Developed Rubber Sole for Prosthesis Applying Tire Technology-Team Bridgestone - Athletes' Challenge", INTERNET CITATION, 14 April 2018 (2018-04-14), pages 1 - 2, XP009534252, Retrieved from the Internet <URL:https://www.bridgestone.co.jp/corporate/news/2018041801.html> [retrieved on 20230727]
- ANONYMOUS: "Supporting Athletes with Newly Developed Rubber Sole for Prosthesis Applying Tire Technology-Team Bridgestone • Athletes' Challenge", JP, pages 1 - 2, XP009534252, Retrieved from the Internet <URL:https://www.bridgestone.co.jp/corporate/news/2018041801.html>

## Description

### TECHNICAL FIELD

The present disclosure relates to a sole of an athletic prosthetic leg.

### BACKGROUND

Conventionally, a prosthetic leg for a competition (hereinafter, simply "athletic prosthetic leg") having a leaf-spring shaped leg portion which extends to a toe side while curving is well-known. A sole which abuts against the ground contact surface is attached to a bottom surface of the ground contact region in the leg portion of the athletic prosthetic leg. Patent literature (PTL) 1 discloses this type of sole of an athletic prosthetic leg. The sole disclosed in PTL 1 corresponds to sporting events such as jogging or running. Further reference is made to disclosures in PTL 2 to PTL 5, NPL 1 and NPL 2. In particular, NPL 2 discloses a rubber sole for a prosthetic leg, the bottom surface of which is provided with grooves for improving grip during running. The sole includes grooves extending in the sole width direction. However, the grooves in the main region and the open groove have substantially the same groove width, and the toe region includes a groove extending to an outer edge of the sole.

### CITATION LIST

### Patent and Non-Patent Literature

PTL 1: JP 2016-150189 A
PTL 2: US 8535390 B1
PTL 3: US 4550510 A
PTL 4: EP 1972223 A1
PTL 5: US D0814160 S
NPL 1: Miles Stuart, "Nike and Sarah Reinersten create new shoe tech for amputee athletes, 16 April 2013, URL: https%3A%2F%2Fwww.pocket-lint.com%2Fgadgets%2Fnews%2Fnike%2F115074-sarah-reinstern-talks-nike-sole%2F
NPL 2: Bridgestone Corporation, "Supporting Athletes with Newly Developed Rubber Sole for Prosthesis Applying Tire Technology-Team Bridgestone - Athletes' Challenge", Internet Citation, 14 April 2018, p1-2, URL:https://www.bridgestone.co.jp/corporate/news/2018041801.html

### SUMMARY

### (Technical Problem)

As a result of intense study, I recognized that there is room for improvement in the anti-slip properties and wear resistance of the sole of an athletic prosthetic leg during running.

The present disclosure aims to provide a sole of an athletic prosthetic leg that can achieve both anti-slip properties and wear resistance during running.

### (Solution to Problem)

A sole of an athletic prosthetic leg of the present disclosure is configured to be attached to a ground contact region of the athletic prosthetic leg, wherein an open groove extending from an outer edge in a sole width direction at least to a central location in the sole width direction is provided on a bottom surface, the bottom surface is divided by the open groove into a toe region at a front side in a sole front-back direction and a main region at a back side in the sole front-back direction, a first groove having a minimum groove width larger than a maximum groove width of the open groove and a second groove extending to an outer edge and having a maximum groove width equal to or less than the maximum groove width of the open groove are not provided in the toe region, and a plurality of third grooves which extend over the entire sole width direction and each of which has a minimum groove width larger than the maximum groove width of the open groove are provided in the main region, and the plurality of third grooves are defined between a plurality of land portions extending over the entire sole width direction in a zigzag shape that is repeatedly convex and concave in the sole front-back direction and arranged at intervals in the sole front-back direction.

### (Advantageous Effect)

According to the present disclosure, a sole of an athletic prosthetic leg that achieves both anti-slip properties and wear resistance during running can be provided.

### BRIEF DESCRIPTION OF THE DRAWINGS

In the accompanying drawings:
FIG 1 is a side view of an athletic prosthetic leg to which a sole according to an embodiment of the present disclosure is attached;
FIG. 2 is an enlarged view of the area near the ground contact region illustrated in FIG. 1;
FIG. 3 is a developed view of the bottom surface of the sole illustrated in FIG. 2;
FIG. 4 is an enlarged view of a portion of FIG. 3; and
FIG. 5 is an enlarged view of a portion of an open groove in FIG. 4.

### DETAILED DESCRIPTION

An embodiment of a sole of an athletic prosthetic leg according to the present disclosure will be described below with reference to the drawings. Members and components that are common across drawings are labeled with the same reference signs.

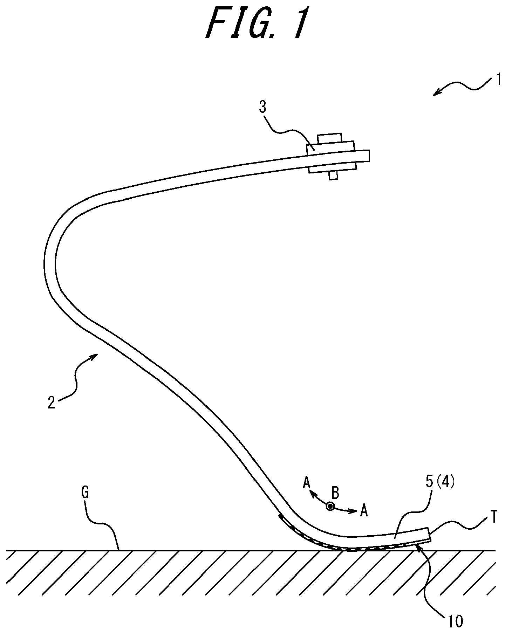

First, an overview of an example of an athletic prosthetic leg to which the sole of an athletic prosthetic leg of the present disclosure is attached will be described. FIG. 1 is a side view of an athletic prosthetic leg 1 as an example of an athletic prosthetic leg to which the sole according to the present disclosure can be attached. The athletic prosthetic leg 1 illustrated in FIG. 1 includes a leaf-spring shaped curved portion 2 and an attachment portion 3 provided at one end of the curved portion 2. The other end of the curved portion 2 is a ground contact portion 5 that contacts a ground contact surface G. In other words, the ground contact region 4 of the athletic prosthetic leg 1 of the present embodiment is configured by the ground contact portion 5 of the curved portion 2. A sole 10 as an embodiment of the present disclosure is attached to the ground contact portion 5 of the curved portion 2. A knee joint, for example, is attached to the attachment portion 3. The wearer can wear the athletic prosthetic leg 1 by, for example, attaching the thigh portion to a socket attached to the knee joint. However, the athletic prosthetic leg 1 illustrated in FIG. 1 is only an example to which the sole according to the present disclosure can be attached, and the configuration thereof is not limited. Although the athletic prosthetic leg 1 illustrated in FIG. 1 has a configuration usable in the case of the wearer's amputation level being a knee disarticulation, the athletic prosthetic leg may, for example, be usable for wearers with different amputation levels, such as lower leg amputation and thigh amputation.

FIG. 1 is a side view of the athletic prosthetic leg 1 in a state in which the wearer of the athletic prosthetic leg 1 is standing upright on a horizontal ground contact surface G. For convenience, the state of the athletic prosthetic leg 1 illustrated in FIG. 1 will be referred to as a "reference state". In the athletic prosthetic leg 1, the end of the curved portion 2 with the attachment portion 3 is referred to as the "attachment side", and the other end of the curved portion 2 without the attachment portion 3 is referred to as the "ground contact side". The toe T of the athletic prosthetic leg 1 refers to the tip of the ground contact region 4. Specifically, the toe T of the athletic prosthetic leg 1 in the present embodiment refers to the tip extending from the attachment side to the ground contact side and terminating at the ground contact side. Furthermore, the heel of the athletic prosthetic leg 1 refers to the base of the ground contact region 4. The sole 10 is attached to the athletic prosthetic leg 1 so as to cover at least the area from the toe T to the heel of the athletic prosthetic leg 1.

Hereinafter, the end of the sole 10 that becomes the toe T side upon attachment to the athletic prosthetic leg 1 will be referred to as the "front side", and the other end that becomes the heel side will be referred to as the "back side". The direction from the front side to the back side of the sole 10 and the direction from the back side to the front side of the sole 10 are together referred to as the "sole front-back direction A". Furthermore, the width direction of the sole orthogonal to the sole front-back direction A is simply referred to as the "sole width direction B".

According to the athletic prosthetic leg 1, a repulsive force can be generated by receiving a reaction force from the ground contact surface G to flex the leaf-spring shaped curved portion 2. The wearer can use this repulsive force to gain propulsion. The curved portion 2 is preferably made using carbon fiber reinforced plastic or the like from the viewpoint of strength and weight reduction. As illustrated in FIG. 1, the sole 10 is attached to the lower surface of the ground contact portion 5 as the ground contact region 4 of the athletic prosthetic leg 1 that contacts the ground contact surface G.

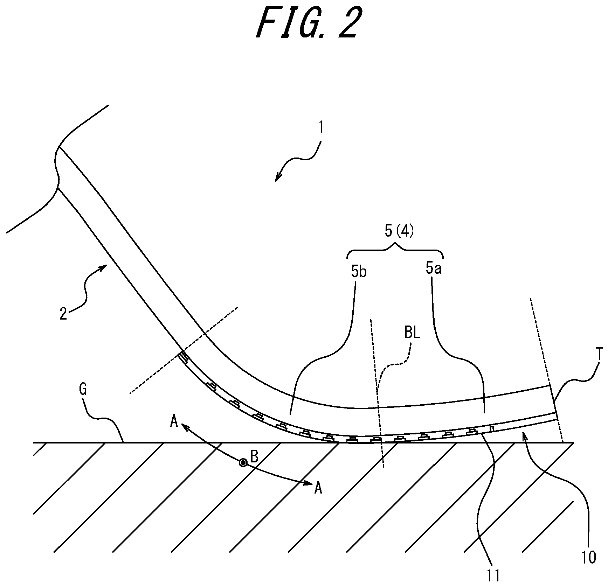

FIG. 2 is an enlarged view of the area near the ground contact region 4 illustrated in FIG. 1. As illustrated in FIGS. 1 and 2, the sole 10 has a shape that follows the extending shape of the ground contact portion 5 as the ground contact region 4. As illustrated in FIG. 2, the ground contact portion 5 as the ground contact region 4 is configured by a curved plate portion that is curved so as to be convex towards the ground contact surface G in a side view. The sole 10 is attached along the bottom surface of the ground contact portion 5 that is convex towards the ground contact surface G.

More specifically, as illustrated in FIG. 2, the ground contact portion 5 as the ground contact region 4 is formed by a continuous plurality of arc sections with different radii of curvature in a side view. Specifically, the ground contact portion 5 in the present embodiment is configured by a first curved plate 5a on the toe T side and a second curved plate 5b on the heel side. In the side view illustrated in FIG. 2, the radius of curvature of the first curved plate 5a is larger than the radius of curvature of the second curved plate 5b. The sole 10 is attached across the lower surface of the first curved plate 5a and the lower surface of the second curved plate 5b.

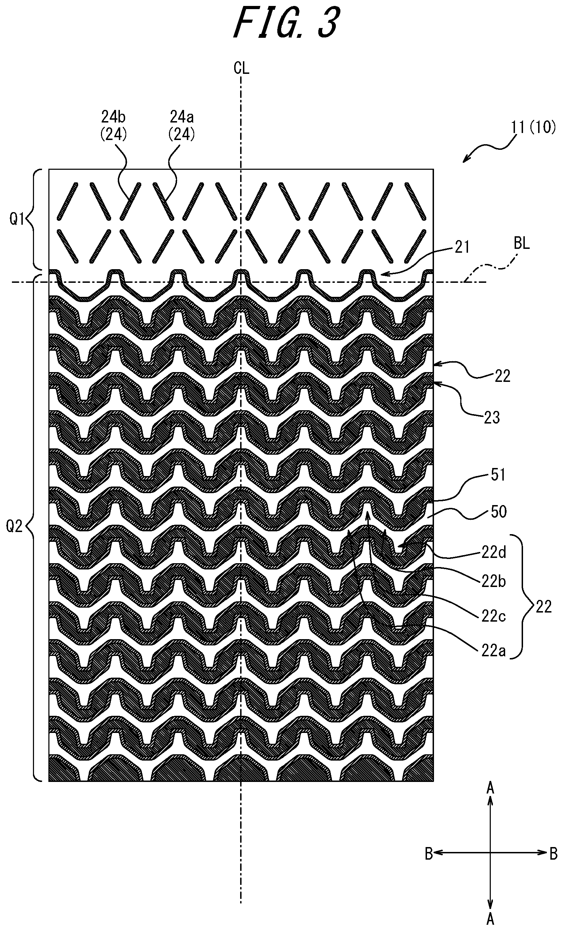

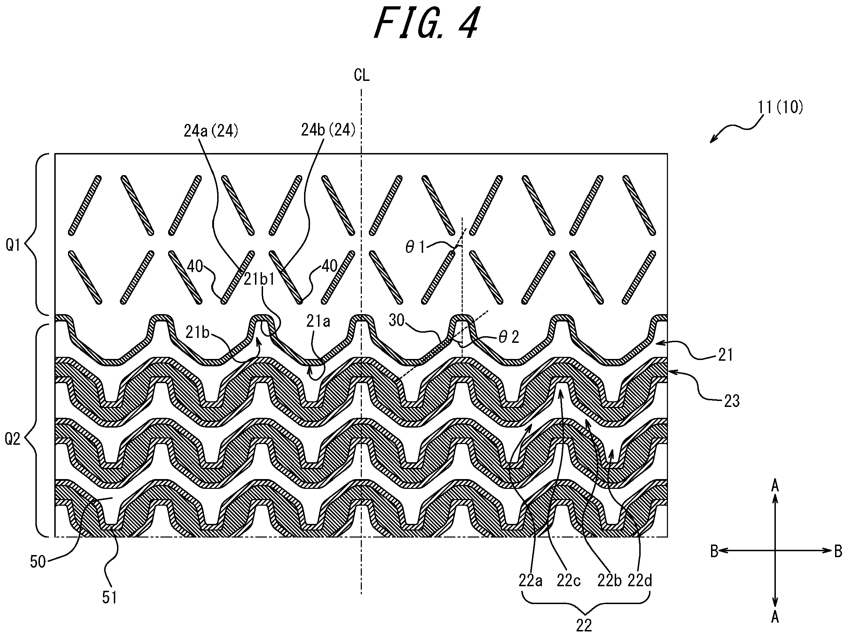

Next, the details of a bottom surface 11, of the sole 10, that abuts against the ground contact surface G are described. FIG. 3 is a developed view of the bottom surface 11 of the sole 10. FIG. 4 is an enlarged view of a portion of FIG. 3.

The front side of the bottom surface 11 of the sole 10 is subjected to a large load during a kick-out movement by the wearer and is therefore prone to wear. On the other hand, the back side of the sole 10 is required to have high anti-slip properties to prevent the wearer from slipping when transitioning from a step-in movement to the kick-out movement. Based on this knowledge, in the bottom surface 11 of the sole 10 illustrated in FIGS. 3 and 4, a region with enhanced wear resistance is disposed on the front side, and an area with an enhanced anti-slip property is arranged on the back side.

The bottom surface 11 illustrated in FIGS. 3 and 4 includes a region with a plurality of grooves 23 extending in the sole width direction B as the region with enhanced anti-slip properties on the back side in the sole front-back direction A. While details are provided below, this region corresponds to a main region Q2 in the present embodiment. The main region Q2 is configured by a plurality of land portions 22 extending over the entire sole width direction B and arranged at intervals in the sole front-back direction A, and a plurality of grooves 23 defined between the plurality of land portions 22 and extending over the entire sole width direction B. In other words, the grooves 23 in the present embodiment refer to portions recessed below a virtual surface taken as a reference and formed by a ground contact top surface 50 of the land portions 22. While details are provided below, the minimum groove width of the grooves 23 is larger than the maximum groove width of an open groove 21, described below. The provision of such grooves 23 can improve the drainage performance and enhance the anti-slip properties. The groove width of the open groove 21 and the grooves 23 refers to the length in the direction orthogonal to the extending direction of each groove. As will be described in detail later, the open groove 21 and the grooves 23 of the present embodiment extend in a zigzag shape. In the case of such zigzag grooves, a zigzag line connecting the center positions in the sole front-back direction A is defined as the extending direction of the groove, and the length in the direction orthogonal to this extending direction is the groove width. A step is provided on the groove wall of the groove 23 in the present embodiment, and a stepped surface 51 facing the ground contact surface G (see FIG. 1 and the like) side is formed. The cross-sectional shape of the groove 23 along the groove width direction is not, however, limited to this shape. For example, the groove 23 may be a groove with a rectangular cross-sectional shape along the groove width direction, with no such step provided in the groove wall.

The land portions 22 of the main region Q2 extend towards the sole width direction B in a zigzag shape that is repeatedly convex and concave in the sole front-back direction A. This configuration can secure the edge component of the land portions 22 as compared to the case of straight land portions. This can improve the water-cutting properties to cut the water film on the ground contact surface G when stepping in, and can enhance the anti-slip properties.

More specifically, each land portion 22 includes a first inclined portion 22a, a second inclined portion 22b, a front convex portion 22c, and a back convex portion 22d.

The first inclined portion 22a extends at an inclination towards one side with respect to the sole front-back direction A. The second inclined portion 22b extends at an inclination towards the other side with respect to the sole front-back direction A. The land portion width of the first inclined portion 22a and the second inclined portion 22b are substantially constant. The land portion width of the first inclined portion 22a and the second inclined portion 22b refers to the length in the direction orthogonal to the extending direction of the first inclined portion 22a and the second inclined portion 22b. The extending direction of the first inclined portion 22a and the second inclined portion 22b is defined as the line connecting the center positions in the sole front-back direction A. The land portion width of the first inclined portion 22a and the second inclined portion 22b in the present embodiment is substantially constant, but this configuration is not limiting. The land portion width may be configured to vary depending on the position in the extending direction.

The front convex portion 22c projects towards the front at the intersection of the first inclined portion 22a and the second inclined portion 22b. The back convex portion 22d projects towards the back at the intersection of the first inclined portion 22a and the second inclined portion 22b. As a result of the land portion 22 including the front convex portion 22c and the back convex portion 22d, the edge component of the land portion 22 can be further increased, and the water-cutting properties to cut the water film on the ground contact surface G can be further enhanced. As a result, the anti-slip properties of the main region Q2 can be further enhanced.

Each land portion 22 is formed by continuous repetition, in the sole width direction B, of a pattern of individual units, each individual unit consisting of the first inclined portion 22a, the front convex portion 22c, the second inclined portion 22b, and the back convex portion 22d, in that order.

As for the positional relationships between the plurality of land portions 22 that are arranged at intervals in the sole front-back direction A, the positions in the sole width direction B are the same for each of the first inclined portions 22a, the front convex portions 22c, the second inclined portions 22b, and the back convex portions 22d. In other words, the positions of the first inclined portions 22a, the front convex portions 22c, the second inclined portions 22b, and the back convex portions 22d of the plurality of land portions 22 are aligned in the sole width direction B so as to line up in a row in the sole front-back direction A. With this configuration, the groove 23 that is defined between two adjacent land portions 22 in the sole front-back direction A also extends towards the sole width direction B in a zigzag shape that is repeatedly convex and concave in the sole front-back direction A.

Furthermore, in two adjacent land portions 22 in the sole front-back direction A, the front convex portion 22c of the land portion 22 on the back side and the back convex portion 22d of the land portion 22 on the front side are at the same position in the sole front-back direction A. In other words, the front convex portion 22c of the land portion 22 on the back side and the back convex portion 22d of the land portion 22 on the front side are at different positions in the sole width direction B but overlap in the sole front-back direction A. This configuration can increase the number of land portions 22 arranged in the sole front-back direction A as compared to a configuration in which adjacent land portions do not overlap in the sole front-back direction. The edge component of the land portion 22 can thus be increased. As a result, the water-cutting properties can be improved, and the anti-slip properties can be enhanced.

Although the plurality of land portions 22 in the present embodiment are all uniformly shaped, this configuration is not limiting. The land portion width of the land portions 22 can be varied according to the position in the sole front-back direction A. Although the plurality of grooves 23 in the present embodiment are all uniformly shaped, this configuration is not limiting. The groove width of the grooves 23 can be varied according to the position in the sole front-back direction A. Furthermore, the ends of the grooves 23 in the present embodiment extend to the outer edges on both sides in the sole width direction B. According to an unclaimed embodiment the ends of the grooves 23 may terminate in the bottom surface 11, or only one of the ends may extend to an outer edge.

The bottom surface 11 illustrated in FIGS. 3 and 4 includes a region without wide grooves, such as the above-described grooves 23, as a region with enhanced wear resistance on the front side in the sole front-back direction A. While details are provided below, this region corresponds to a toe region Q1 in the present embodiment.

In the toe region Q1 of the present embodiment, a plurality of terminating grooves 24 that terminate within the toe region Q1 without extending to an outer edge of the bottom surface 11 is provided. The toe region Q1 of the present embodiment only includes the plurality of terminating grooves 24, and no other grooves are provided. While details are provided below, the maximum groove width of the terminating grooves 24 is equal to or less than the maximum groove width of the open groove 21. The groove width of the terminating grooves 24 refers to the length in the direction orthogonal to the extending direction of the terminating grooves 24, like the above-described groove width of the open groove 21 and the grooves 23.

The terminating groove 24 is a narrow groove that closes, so that the groove walls abut against and support each other, when the wearer contacts the ground while running. Such a narrow groove is called a "sipe". Such a sipe is defined as a groove with a maximum groove width equal to or less than 1/2 of the groove depth in the sole thickness direction. The maximum groove width of the terminating groove 24 in the present embodiment is set to 1 mm or less. By contrast, the grooves 23 in the above-described main region Q2 are thick grooves that do not close, i.e., in which the groove walls remain separated, when the wearer contacts the ground while running. Such a thick groove is defined as a groove with a minimum groove width greater than 1/2 of the groove depth in the sole thickness direction. The minimum groove width of the grooves 23 in the present embodiment is set to be wider than 1 mm.

In this way, the bottom surface 11 of the sole 10 of the present embodiment includes a region with enhanced wear resistance located on the front side and a region with enhanced anti-slip properties located on the back side. This configuration can suppress wear at the toe T side to endow the sole 10 with high durability. The anti-slip properties on the heel side can also be enhanced, yielding a highly safe sole 10 whose wearer is less likely to slip while running.

As a result of further intense study, I recognized a further problem in that a step in rigidity tends to occur between the above-described region with high wear resistance at the front side and the above-described region with high anti-slip properties at the back side, making this position prone to uneven wear.

To address this issue, the open groove 21 is provided on the bottom surface 11 of the sole 10 illustrated in FIGS. 3 and 4, between the above-described region with high wear resistance and the above-described region with high anti-slip properties. In other words, the bottom surface 11 is divided by the open groove 21 into the toe region Q1 on the front side in the sole front-back direction A and the main region Q2 on the back side in the sole front-back direction A. The open groove 21 extends from an outer edge in the sole width direction B to at least a central location CL in the sole width direction B. The central location CL in the sole width direction B is defined by the set of midpoints in the sole width direction B at each position in the sole front-back direction A. The toe region Q1 and the main region Q2 are regions that satisfy at least the following three requirements.
(1) A groove having a minimum groove width larger than the maximum groove width of the open groove 21 is not provided in the toe region Q1.
(2) A groove extending to an outer edge and having a maximum groove width equal to or less than the maximum groove width of the open groove 21 is not provided in the toe region Q1.
(3) A groove having a minimum groove width larger than the maximum groove width of the open groove 21 is provided in the main region Q². Additionally, or as an alternative according to an unclaimed embodiment, the negative ratio of the main region Q2 is greater than the negative ratio of the toe region Q1.

In the toe region Q1 illustrated in FIGS. 3 and 4, only the terminating grooves 24, whose maximum groove width is equal to or less than the maximum groove width of the open groove 21, are provided. That is, a groove having a minimum groove width larger than the maximum groove width of the open groove 21 is not provided in the toe region Q1. Therefore, the toe region Q1 illustrated in FIGS. 3 and 4 satisfies requirement (1).

The terminating grooves 24 do not extend to the outer edge of the bottom surface 11, do not open to the open groove 21, and terminate within the bottom surface 11. Like the terminating grooves 24, the open groove 21 of the present embodiment is configured as the above-described sipe. That is, the terminating grooves 24, whose maximum groove width is equal to or less than the maximum groove width of the open groove 21, are provided in the toe region Q1. However, as described above, the terminating grooves 24 are not grooves that extend to the outer edges of the bottom surface 11. Therefore, a groove extending to an outer edge and having a maximum groove width equal to or less than the maximum groove width of the open groove 21 is not provided in the toe region Q1. In other words, the toe region Q1 illustrated in FIGS. 3 and 4 also satisfies requirement (2).

Furthermore, the grooves 23 are provided in the main region Q2 illustrated in FIGS. 3 and 4. As described above, the grooves 23 are not sipes, and the minimum groove width of the grooves 23 is larger than the maximum groove width of the open groove 21. In other words, a groove having a minimum groove width larger than the maximum groove width of the open groove 21 is provided in the main region Q2. Therefore, the main region Q2 illustrated in FIGS. 3 and 4 satisfies requirement (3).

The main region Q2 illustrated in FIGS. 3 and 4 also satisfies the portion of requirement (3) stating that "the negative ratio of the main region Q2 is greater than the negative ratio of the toe region Q1". The "negative ratio" refers to the proportion of concave portions among bottom surface concave and convex portions. Specifically, the negative ratio of the toe region Q1 in the present embodiment refers to the ratio of the area occupied by all the terminating grooves 24 illustrated in FIG. 3 to the total area of the toe region Q1 illustrated in FIG. 3. The negative ratio of the main region Q2 in the present embodiment refers to the ratio of the area occupied by all the grooves 23 illustrated in FIG. 3 to the total area of the main region Q2 illustrated in FIG. 3.

As described above, the bottom surface 11 of the sole 10 of the present embodiment includes the toe region Q1 and the main region Q2 that satisfy all of the above requirements (1), (2), and (3). As a result of the open groove 21, which extends over a predetermined range in the sole width direction B, being provided between the toe region Q1 as a region with high wear resistance and the main region Q2 as a region with high anti-slip properties, the step in rigidity between the toe region Q1 and the main region Q2 can be reduced. In particular, the step in rigidity tends to increase due to the difference between the toe region Q1 and the main region Q2 regarding the presence of thick grooves such as the grooves 23. Therefore, even when the open groove 21 that is narrower than the grooves 23 is provided, the configuration in which the open groove 21 extends to the outer edge can secure deformation properties and can mitigate the aforementioned step in rigidity. Consequently, the occurrence of uneven wear can be suppressed at the position between the area with high wear resistance and the area with high anti-slip properties. Furthermore, by the open groove 21 being provided, water-cutting properties can be achieved by the edge component of the open groove 21 while maintaining the desired rigidity that mitigates the aforementioned step in rigidity.

The toe region Q1 of the present embodiment includes the terminating grooves 24 extending in a straight line in a bottom surface view (see FIG. 3 and the like) but may instead be configured without any grooves.

Next, further details of the bottom surface 11 of the sole 10 of the present embodiment are provided.

As described above, the open groove 21 is configured to extend from an outer edge in the sole width direction B to at least the central location CL in the sole width direction B. However, the open groove 21 preferably extends from one outer edge to the other outer edge in the sole width direction B, as in the present embodiment. In this way, the variation in rigidity in the sole width direction B can be suppressed, and the occurrence of a step in rigidity in the sole width direction B can be suppressed. The occurrence of uneven wear can thereby be suppressed in a portion in the sole width direction B. Variation in the water-cutting properties in the sole width direction B can also be suppressed.

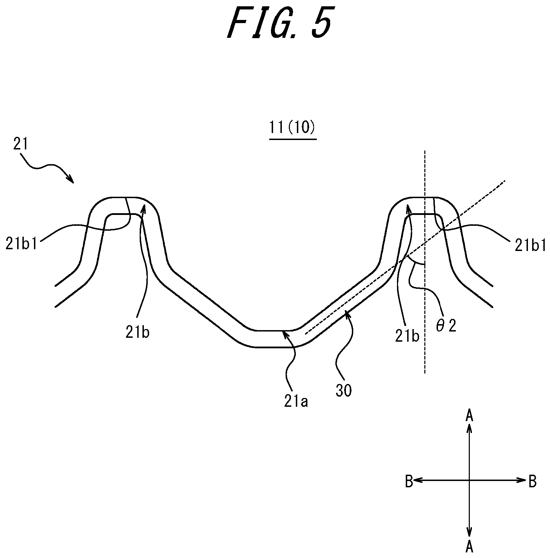

Furthermore, as illustrated in FIG. 4, the open groove 21 of the present embodiment extends towards the sole width direction B in a zigzag shape that is repeatedly convex and concave in the sole front-back direction. By the open groove 21 with this shape being provided, convex wall portions 21a and concave wall portions 21b are formed in the groove wall of the open groove 21. FIG. 5 is a further enlarged view of a portion of the open groove 21 illustrated in FIG. 4. As illustrated in FIG. 5, the convex wall portion 21a is a portion, of the groove wall located on the front side of the open groove 21, that projects towards the back side, i.e., the main region Q2 side (lower side in FIG. 5). As illustrated in FIG. 5, the concave wall portion 21b is a portion, of the groove wall located on the front side of the open groove 21, that is recessed towards the toe region Q1 side (upper side in FIG. 5).

The groove wall of the open groove 21 in the present embodiment includes a plurality of the above-described convex wall portions 21a. The groove wall of the open groove 21 in the present embodiment also includes a plurality of the above-described concave wall portions 21b. The convex wall portions 21a and the concave wall portions 21b are arranged alternately in the sole width direction B. More specifically, each concave wall portion 21b in the present embodiment is defined at the position between two convex wall portions 21a, adjacent in the sole width direction B. The number and shape of the convex wall portions 21a and concave wall portions 21b are not limited to the number and shape of the present embodiment.

By the groove wall of the open groove 21 including the above-described convex wall portions 21a, a water-cutting effect of cutting a water film on the ground contact surface G (see FIG. 1) with the apex of the convex wall portion 21a during running can be achieved. This can further enhance the anti-slip properties.

As illustrated in FIG. 4, the groove wall of the open groove 21 includes the above-described concave wall portions 21b. As described above, the plurality of terminating grooves 24 is provided in the toe region Q1 of the present embodiment. Each terminating groove 24 extends in the sole front-back direction A. Here, as illustrated in FIG. 4, end portions 40 on the main region Q2 side of the terminating grooves 24, among the plurality of terminating grooves 24, that are closest to the open groove 21 in the sole front-back direction A are provided at different positions in the sole width direction B than concave bottoms 21b1 of the concave wall portions 21b. In other words, the end portions 40 on the main region Q2 side of the terminating grooves 24, among the plurality of terminating grooves 24, that are closest to the open groove 21 in the sole front-back direction A and the concave bottoms 21b1 of the concave wall portions 21b are formed to be at different positions in the sole width direction B. More specifically, the end portions 40 on the main region Q2 side of the terminating grooves 24, among the plurality of terminating grooves 24 of the present embodiment, that are closest to the open groove 21 in the sole front-back direction A are each positioned between two adjacent concave wall portions 21b in the sole width direction B. The position in the sole width direction B of the end portions 40 of the terminating grooves 24 on the main region Q2 side thus differ from the positions of the concave bottoms 21b1 of the concave walls 21b in the sole width direction B. As compared to a configuration in which these positions in the sole width direction B are the same, the distance between the terminating grooves 24 and the open groove 21 in the sole front-back direction A can thereby be prevented from varying depending on the position in the sole width direction B. This can suppress variation in the rigidity in the sole width direction B and suppress the occurrence of uneven wear.

The shape of the land portion defined between the open groove 21 of the present embodiment and the groove 23 farthest to the front in the main region Q2 is the same as that of the land portion 22 in the main region Q2, but this configuration is not limiting. The maximum land portion width of the land portion defined between the open groove 21 and the groove 23 farthest to the front in the main region Q2 is, however, preferably equal to or less than the maximum land portion width of the land portion 22. This configuration can suppress an excessive increase in rigidity at the position adjacent to the back side of the open groove 21. This configuration can also suppress an excessive decrease in the drainage performance at the position adjacent to the back side of the open groove 21.

As illustrated in FIG. 4, the open groove 21 and the terminating grooves 24 in the present embodiment do not overlap at all in the sole front-back direction A and are not lined up in a row in any portion in the sole width direction B. The toe region Q1 of the present embodiment includes a region with no groove extending in a straight line in the sole width direction B at a position between the open groove 21 and the terminating grooves 24 in the sole front-back direction A, as illustrated in FIG. 4. This configuration can prevent the rigidity in the toe region Q1 from being reduced due to the open groove 21.

In the present embodiment, the open groove 21 is configured in a zigzag shape to form the above-described convex wall portions 21a and concave wall portions 21b, but the shape of the open groove 21 is not limited to a zigzag shape. Therefore, the convex wall portions 21a and concave wall portions 21b may be formed on the groove wall of an open groove 21 that has a different shape. However, the plurality of convex wall portions 21a and the plurality of concave wall portions 21b can be easily formed if the open groove 21 is zigzag-shaped as in the present embodiment.

Furthermore, the terminating grooves 24 of the present embodiment extend at an inclination relative to the sole front-back direction A in a bottom surface view (see FIG. 4 and the like). Specifically, the terminating grooves 24 of the present embodiment are inclined at an angle of θ1 relative to the sole front-back direction A. The open groove 21 of the present embodiment includes inclined extending portions 30 that extend at a greater angle θ2 than the terminating grooves 24 relative to the sole front-back direction A in a bottom surface view (see FIG. 4 and the like). The inclined extending portions 30 of the present embodiment extend in a straight line in a bottom surface view (see FIG. 4 and the like). Even if the terminating grooves 24 are provided in the toe region Q1, a configuration that bends and deforms out of the plane more easily at the position of the open groove 21 than at the position of the toe region Q1 can be achieved by the angle relationship between the angle θ1 of the terminating grooves 24 and the angle θ2 of the inclined extension portions 30 of the open groove 21 being set to the aforementioned relationship. In other words, adoption of the aforementioned angular relationship ensures that the sole 10 follows the shape of the ground contact portion 5 of the athletic prosthetic leg 1, regardless of whether terminating grooves 24 are provided in the toe region Q1.

As described above, the plurality of terminating grooves 24 is provided in the toe region Q1 of the present embodiment. The plurality of terminating grooves 24 includes a first terminating groove 24a that is inclined towards one side in the sole width direction B while extending towards the front side in the sole front-back direction A, and a second terminating groove 24a that is inclined towards the other side in the sole width direction B while extending towards the front side in the sole front-back direction A. The first terminating groove 24a and the second terminating groove 24b are linearly symmetrical about an axis of symmetry extending in the sole front-back direction A in the bottom surface view of FIG. 4. The first terminating groove 24a and the second terminating groove 24b are arranged alternately in the sole width direction B. In the toe region Q1 of the present embodiment, two widthwise rows in which first terminating groove 24a and the second terminating groove 24b are arranged alternately in the sole width direction B are provided at different positions in the sole front-back direction A. The first terminating grooves 24a in one of the two widthwise rows are adjacent in the sole front-back direction A to the second terminating grooves 24b in the other widthwise row. The second terminating grooves 24b in one of the two widthwise rows are adjacent in the sole front-back direction A to the first terminating grooves 24a in the other widthwise row.

The sole 10 of the present embodiment is configured to have a substantially uniform thickness. Specifically, in the sole 10 of the present embodiment, the thickness in the toe region Q1 is substantially equal to the thickness in the main region Q2. In the present embodiment, the thickness in the toe region Q1 of the sole 10 refers to the thickness at a position where no terminating groove 24 is provided. Also, in the present embodiment, the thickness in the main region Q2 of the sole 10 refers to the thickness at the position of the ground contact top surface 50 of the land portion 22 (see FIGS. 3 and 4).

Next, the details of the sole 10 as attached to the athletic prosthetic leg 1 are provided. As illustrated in FIG. 2, the sole 10 is attached to the ground contact portion 5 as the ground contact region 4 of the athletic prosthetic leg 1. The sole 10 is attached to the ground contact portion 5 by covering the lower surface of the ground contact portion 5 on the ground contact surface G side of the curved portion 2 and being wrapped around to the upper surface of the ground contact portion 5 and fixed. The portion of the sole 10 that wraps around the ground contact portion 5 is omitted from FIGS. 1 to 5.

As described above, the ground contact portion 5 of the present embodiment is configured by the first curved plate 5a and the heel-side second curved plate part 5b that have different radii of curvature in a side view (see FIG. 2). As described above, in the side view illustrated in FIG. 2, the radius of curvature of the first curved plate 5a is larger than the radius of curvature of the second curved plate 5b. The radius of curvature of the first curved plate 5a may, however, be equal to or less than the radius of curvature of the second curved plate 5b. In the case in which the radius of curvature of the first curved plate 5a and the second curved plate 5b are different, the boundary line BL between the first curved plate portion 5a and the second curved plate portion 5b is disposed at a position overlapping the open groove 21 on the bottom surface 11 of the sole 10, as illustrated in FIG. 3. In this way, even if the radius of curvature changes at the boundary line BL on the ground contact portion 5, the sole 10 can easily be made to follow the ground contact portion 5.

The sole of an athletic prosthetic leg according to the present disclosure is not limited to the specific configurations described in the above embodiments. Various modifications and changes may be made without departing from the scope of the claims. In the above embodiment, the grooves 23 are provided in the main region Q2 on the bottom surface 11 of the sole 10. However, according to unclaimed embodiments, as long as the negative ratio of the main region Q2 is greater than the negative ratio of the toe region Q1, concave portions other than grooves, such as dimples, may be adopted. In such unclaimed embodiments, the concave portions may be configured to penetrate or not to penetrate to the outer edge of the bottom surface 11. However, as in the present embodiment, in the claimed invention, the grooves 23 are adopted as the concave portions and configured to penetrate to the outer edge of the bottom surface 11. This configuration can further enhance the drainage performance of the main region Q2 of the bottom surface 11 and further enhance the anti-slip properties of the main region Q2. According to unclaimed embodiments, as long as the negative ratio of the main region Q2 is greater than the negative ratio of the toe region Q1, the main region Q2 may have a plurality of convex portions disposed thereon. In this case, the positions in the main region Q2 at which no convex portions are provided in the main region Q2 become concave portions in the main region Q2.

### INDUSTRIAL APPLICABILITY

The present disclosure relates to a sole of an athletic prosthetic leg.

### REFERENCE SIGNS LIST

- 1: Athletic prosthetic leg
- 2: Curved portion
- 3: Attachment portion
- 4: Ground contact region
- 5: Ground contact portion
- 5a: First curved plate
- 5b: Second curved plate
- 10: Sole
- 11: Bottom surface
- 21: Open groove
- 21a: Convex wall portion
- 21b: Concave wall portion
- 21b1: Concave bottom
- 22: Land portion
- 22a: First inclined portion
- 22b: Second inclined portion
- 22c: Front convex portion
- 22d: Back convex portion
- 23: Groove
- 24: Terminating groove
- 24a: First terminating groove
- 24b: Second terminating groove
- 30: Inclined extending portion
- 50: Ground contact top surface
- 51: Stepped surface
- A: Sole front-back direction
- B: Sole width direction
- BL: Boundary line
- CL: Central location
- G: Ground contact surface
- Q1: Toe region
- Q2: Main region
- T: Toe
- θ1: Angle of terminating groove relative to sole front-back direction
- θ2: Angle of inclined extending portion of open groove relative to sole front-back direction

## Claims

1. A sole (10) of an athletic prosthetic leg (1), the sole (10) being configured to be attached to a ground contact region (2) of the athletic prosthetic leg (1), wherein
an open groove (21) extending from an outer edge in a sole width direction (B) at least to a central location (CL) in the sole width direction (B) is provided on a bottom surface (11),
the bottom surface (11) is divided by the open groove (21) into a toe region (Q1) at a front side in a sole front-back direction (A) and a main region (Q2) at a back side in the sole front-back direction (A),
a first groove having a minimum groove width larger than a maximum groove width of the open groove (21) and a second groove extending to an outer edge and having a maximum groove width equal to or less than the maximum groove width of the open groove (21) are not provided in the toe region (Q1),
a plurality of third grooves (23) which extend over the entire sole width direction (B) and each of which has a minimum groove width larger than the maximum groove width of the open groove (21) are provided in the main region (Q2), and
the plurality of third grooves (23) are defined between a plurality of land portions (22) extending over the entire sole width direction (B) in a zigzag shape that is repeatedly convex and concave in the sole front-back direction (A) and arranged at intervals in the sole front-back direction (A).

2. The sole (10) of the athletic prosthetic leg (1) of claim 1, wherein the open groove (21) extends from one outer edge to another outer edge in the sole width direction (B).

3. The sole (10) of the athletic prosthetic leg (1) of claim 1 or 2, wherein a groove wall of the open groove (21) includes a plurality of convex wall portions (21a) projecting towards the main region (Q2).

4. The sole (10) of the athletic prosthetic leg (1) of claim 3, wherein
a plurality of terminating grooves (24) is provided in the toe region (Q1), each terminating groove (24) having a maximum groove width equal to or less than the maximum groove width of the open groove (21) and terminating within the toe region (Q1) without extending to an outer edge,
the groove wall of the open groove (21) includes a plurality of concave wall portions (21b) recessed towards the toe region (Q1), and
the plurality of terminating grooves (24) extends in the sole front-back direction (A), and end portions on the main region side of terminating grooves, among the plurality of terminating grooves (24), that are closest to the open groove (21) in the sole front-back direction (A) are provided at different positions in the sole width direction (B) than the concave wall portions (21b).

5. The sole (10) of the athletic prosthetic leg (1) of claim 4, wherein the open groove (21) extends along the sole width direction (B) in a zigzag shape that is repeatedly convex and concave in the sole front-back direction (A).

6. The sole (10) of the athletic prosthetic leg (1) of claim 4 or 5, wherein
the terminating grooves (24) extend at an inclination relative to the sole front-back direction (A), and
the open groove (21) includes an inclined extending portion (30) that extends at a greater angle than the terminating grooves (24) relative to the sole front-back direction (A).

## Patentansprüche

1. Sohle (10) einer Laufprothese (1), wobei die Sohle (10) so konfiguriert ist, dass sie an einem Bodenkontaktbereich (2) der Laufprothese (1) befestigt werden kann, wobei
eine offene Rille (21), die sich von einer Außenkante in einer Sohlenbreitenrichtung (B) mindestens bis zu einer zentralen Stelle (CL) in der Sohlenbreitenrichtung (B) erstreckt, auf einer Unterseite (11) bereitgestellt ist,
die Unterseite (11) durch die offene Rille (21) in einen Zehenbereich (Q1) an der Vorderseite in einer Sohlenlängsrichtung (A) und einen Hauptbereich (Q2) an der Rückseite in der Sohlenlängsrichtung (A) unterteilt ist,
eine erste Rille, die eine Mindestrillenbreite aufweist, die größer als eine maximale Rillenbreite der offenen Rille (21) ist, und eine zweite Rille, die sich bis zu einer Außenkante erstreckt und deren maximale Rillenbreite gleich oder kleiner als die maximale Rillenbreite der offenen Rille (21) ist, im Zehenbereich (Q1) nicht bereitgestellt sind,
eine Vielzahl von dritten Rillen (23), die sich über die gesamte Sohlenbreitenrichtung (B) erstreckt und deren minimale Rillenbreite jeweils größer als die maximale Rillenbreite der offenen Rille (21) im Hauptbereich (Q2) bereitgestellt ist, und
die Vielzahl von dritten Rillen (23) zwischen einer Vielzahl von Stegabschnitten (22) definiert ist, die sich über die gesamte Sohlenbreitenrichtung (B) erstreckt und in einer Zickzackform angeordnet ist, die in Sohlenlängsrichtung (A) wiederholt konvex und konkav ist, und in Sohlenlängsrichtung (A) in Abständen angeordnet ist.

2. Sohle (10) der Laufprothese (1) nach Anspruch 1, wobei sich die offene Rille (21) von einer Außenkante zur anderen Außenkante in der Sohlenbreitenrichtung (B) erstreckt.

3. Sohle (10) der Laufprothese (1) nach Anspruch 1 oder 2, wobei eine Rillenwand der offenen Rille (21) mehrere konvexe Wandabschnitte (21a) beinhaltet, die in Richtung des Hauptbereichs (Q2) vorragen.

4. Sohle (10) der Laufprothese (1) nach Anspruch 3,
wobei
eine Vielzahl von Abschlussrillen (24) im Zehenbereich (Q1) bereitgestellt sind, wobei jede Abschlussrille (24) eine maximale Rillenbreite aufweist, die gleich oder kleiner als die maximale Rillenbreite der offenen Rille (21) ist und innerhalb des Zehenbereichs (Q1) endet, ohne sich bis zu einer Außenkante zu erstrecken,
die Rillenwand der offenen Rille (21) eine Vielzahl von konkavem Wandabschnittem (21b) beinhaltet, die in Richtung des Zehenbereichs (Q1) zurückgesetzt ist, und
die Vielzahl von Abschlussrillen (24) sich in der Sohlenlängsrichtung (A) erstreckt und Endabschnitte auf der Hauptbereichsseite der Sohle von Abschlussrillen von den mehreren Abschlussrillen (24), die der offenen Rille (21) in der Sohlenlängsrichtung (A) am nächsten sind, an anderen Positionen in der Sohlenbreitenrichtung (B) bereitgestellt sind als die konkaven Wandabschnitte (21b).

5. Sohle (10) der Laufprothese (1) nach Anspruch 4, wobei sich die offene Rille (21) entlang der Sohlenbreitenrichtung (B) in Zickzackform erstreckt, die in der Sohlenlängsrichtung (A) wiederholt konvex und konkav ist.

6. Sohle (10) der Laufprothese (1) nach Anspruch 4 oder 5, wobei
die Abschlussrillen (24) sich in einer Neigung relativ zu der Sohlenlängsrichtung (A) erstrecken und
die offene Rille (21) einen geneigten Verlängerungsabschnitt (30) beinhaltet, der sich in einem größeren Winkel als die Abschlussrillen (24) relativ zu der Sohlenlängsrichtung (A) erstreckt.

## Revendications

1. Semelle (10) d'une prothèse de jambe pour la pratique d'athlétisme (1), la semelle (10) étant configurée pour être fixée à une zone de contact au sol (2) de la prothèse de jambe pour la pratique d'athlétisme (1), dans laquelle
une rainure ouverte (21) s'étendant d'un bord extérieur dans le sens de la largeur de la semelle (B) au moins jusqu'à un emplacement central (CL) dans le sens de la largeur de la semelle (B) est prévue sur une surface inférieure (11),
la surface inférieure (11) est divisée par la rainure ouverte (21) en une zone d'orteils (Q1) sur le côté avant dans le sens avant-arrière de la semelle (A) et une zone principale (Q2) sur le côté arrière dans le sens avant-arrière de la semelle (A),
une première rainure présentant une largeur de rainure minimale supérieure à la largeur de rainure maximale de la rainure ouverte (21) et une deuxième rainure s'étendant jusqu'à un bord extérieur et présentant une largeur de rainure maximale inférieure ou égale à la largeur de rainure maximale de la rainure ouverte (21) ne sont pas prévues dans la zone d'orteils (Q1).
une pluralité de troisièmes rainures (23) qui s'étendent sur toute la largeur de la semelle dans le sens (B) et dont chacune présente une largeur de rainure minimale supérieure à la largeur de rainure maximale de la rainure ouverte (21) sont prévues dans la zone principale (Q2), et
la pluralité de troisièmes rainures (23) sont définies entre une pluralité de parties terrestres (22) s'étendant sur toute la largeur de la semelle dans le sens (B) en une forme en zigzag qui est convexe et concave de manière répétée dans le sens avant-arrière de la semelle (A) et agencée à intervalles dans le sens avant-arrière de la semelle (A).

2. Semelle (10) de la prothèse de jambe pour la pratique d'athlétisme (1) selon la revendication 1, dans laquelle la rainure ouverte (21) s'étend d'un bord extérieur jusqu'à un autre bord extérieur dans le sens de la largeur de la semelle (B).

3. Semelle (10) de la prothèse de jambe pour la pratique d'athlétisme (1) selon la revendication 1 ou 2, dans laquelle une paroi de rainure de la rainure ouverte (21) inclut une pluralité de parties de paroi convexes (21a) faisant saillies vers la zone principale (Q2).

4. Semelle (10) de la prothèse de jambe pour la pratique d'athlétisme (1) selon la revendication 3,
dans laquelle
une pluralité de rainures terminales (24) est prévue dans la zone d'orteils (Q1), chaque rainure terminale (24) présentant une largeur de rainure maximale inférieure ou égale à la largeur de rainure maximale de la rainure ouverte (21) et se terminant dans la zone d'orteils (Q1) sans s'étendre jusqu'à un bord extérieur,
la paroi de rainure de la rainure ouverte (21) inclut une pluralité de parties de paroi concaves (21b) en retrait vers la zone d'orteils (Q1), et
la pluralité de rainures terminales (24) s'étend dans le sens avant-arrière de la semelle (A), et les parties d'extrémité sur le côté de la zone principale des rainures terminales, parmi la pluralité de rainures terminales (24), qui sont les plus proches de la rainure ouverte (21) dans le sens avant-arrière de la semelle (A) sont prévues à des positions différentes dans le sens de la largeur de la semelle (B) de celles des parties de paroi concaves (21b).

5. Semelle (10) de la prothèse de jambe pour la pratique d'athlétisme (1) selon la revendication 4, dans laquelle la rainure ouverte (21) s'étend dans le sens de la largeur de la semelle (B) en une forme en zigzag qui est convexe et concave de manière répétée dans le sens avant-arrière de la semelle (A).

6. Semelle (10) de la prothèse de jambe pour la pratique d'athlétisme (1) selon la revendication 4 ou 5, dans laquelle
les rainures terminales (24) s'étendent selon une inclinaison par rapport au sens avant-arrière de la semelle (A), et
la rainure ouverte (21) inclut une partie s'étendant de manière inclinée (30) qui s'étend à un angle plus grand que celui des rainures terminales (24) par rapport au sens avant-arrière de la semelle (A).
